# EUROPEAN PATENT SPECIFICATION

(11) **EP 3 768 149 B1**
(45) Date of publication and mention of the grant of the patent: **01.10.2025**
(21) Application number: 19709872.6
(22) Date of filing: 04.03.2019
(51) Int. Cl.: A61B 5/00, A61C 15/00, A61C 17/02

(54) **MEASUREMENT ELEMENTS FOR ORAL CARE DEVICES**
MESSELEMENTE FÜR MUNDPFLEGEGERÄTE
ÉLÉMENTS DE MESURE POUR DISPOSITIFS DE SOINS BUCCAUX

(30) Priority: 19.03.2018 US 201862644860 P
(43) Date of publication of application: 27.01.2021
(73) Proprietor: Koninklijke Philips N.V., 5656 AG Eindhoven (NL)
(72) Inventor: SCHEFFERS, Lucas Petrus Henricus, 5656 AE Eindhoven (NL); VAN DEN BIJGAART, Adrianus Wilhelmus Dionisius, Maria, 5656 AE Eindhoven (NL); DEANE, Steven Charles, 5656 AE Eindhoven (NL); VERMEULEN, Olaf Thomas Johan Antonie, 5656 AE Eindhoven (NL)
(74) Representative: Philips Intellectual Property & Standards
(86) International application number: PCT/EP2019/055324
(87) International publication number: WO 2019/179757

(56) References cited:
- US-A1- 2007 015 112
- US-A1- 2008 209 650
- US-A1- 2015 327 668
- US-A1- 2016 220 013

## Description

### FIELD OF THE INVENTION

The invention relates generally to oral care and, in particular, to measurement elements for oral care devices.

### BACKGROUND OF THE INVENTION

Within a person's oral cavity, the gingiva (i.e. the gum tissue) may hold valuable information regarding the person's health, particularly their oral health. For example, gingivitis, which manifests itself as an inflammation of the gingiva, can lead to irreversible periodontitis (also referred to as gum disease) if not treated early. Therefore, it is important to detect gingivitis as soon as possible after its onset. Gingivitis predominantly originates in the interproximal space (i.e. the space between teeth in the oral cavity) because these areas tend to be difficult to clean of oral biofilms. Therefore, examination of the gingiva to detect signs of gingivitis tends to be carried out at the interproximal gingiva (also referred to as the papilla).

Teeth range in size both within a single person's oral cavity and between people. For example, the front incisors are typically relatively long and have a relatively flat surface, while the molars are typically relatively wide, with a more rounded surface. In addition, the orientation of teeth and the spacing between adjacent teeth may vary greatly from person to person. Therefore, the gingival papilla between two adjacent teeth may also have a large range of shapes and sizes.

Devices may be used to detect gingivitis within a person's mouth. Such a device may be able to detect gingivitis if a sensing portion of the device is positioned in a particular position relative to the gingival surface, for example with a sensor of the device gently touching the gingival surface. However, contact between the device and the gingival surface can result in altered physiological parameters, for example due to the expulsion of blood from the gingival tissue. On the other hand, if a gap exists between the sensor and the gingival tissue, then a reduced sensing performance may result due to reflections, the spreading of light from the device, and so on. Thus, maintaining an optimum position and orientation of such a sensing device within an oral cavity can pose a significant challenge to a user, and incorrect positioning can lead to gingivitis going undetected.

Therefore, there is a need for a device which can be more easily positioned and oriented within the oral cavity, to allow for this accurate sensing of the gingival tissue.

US 2008/209650 A1 discloses oral hygiene devices. US 2016/220013 A1 discloses a short wavelength visible light-emitting toothbrush with an electronic signal interlock control. US 2007/015112 A1 discloses a teeth whitening apparatus.

### SUMMARY OF THE INVENTION

In order to address at least some of the issues mentioned above, embodiments in the present disclosure provide a device which can be used to perform measurements within a person's oral cavity, and which can be positioned and oriented in an easy, intuitive manner. The structure of the devices disclosed herein is such that a user can position the sensing portion of the device appropriately relative to the gingiva within their oral cavity so that a measurement can be made at an intended location, without the need for the user to see the exact position of the measurement device in their mouth.

According to a first aspect, the invention provides a measurement element for an oral care device, the measurement element comprising an elongate ridge element configured to engage an interproximal region within an oral cavity; and a first sensing region located in a first end portion of the elongate ridge element configured to acquire data from gingival tissue within the oral cavity.

The inclusion of an elongate ridge element as part of the measurement element allows the measurement element to "slot" into an intended position for taking a measurement (e.g. acquiring data) from the gingival tissue between two adjacent teeth in the oral cavity. If the elongate ridge element is aligned with the interproximal region between the adjacent teeth (the interproximal region is also generally elongate in shape), then the ridge element will fall into place, and a user positioning the measurement element within the oral cavity may be able to determine when the measurement element is in the appropriate position for taking measurements (e.g. by the sound of the ridge element lodging into position, or by the restriction of further lateral movement of the measurement element once the ridge element has engaged the interproximal region).

In some embodiments, the measurement element may comprise a first protrusion extending from the first end portion of the elongate ridge element. The first sensing region may be located in a wall of the first protrusion.

The measurement element may, in some embodiments, comprise a second sensing region located in a second end portion of the elongate ridge element for acquiring data from gingival tissue within the oral cavity.

In some embodiments, the measurement element may comprise a second protrusion extending from a second end portion of the elongate ridge element. The second sensing region may be located in a wall of the second protrusion.

Each sensing region may comprise a plurality of sensor windows. In some embodiments, the plurality of sensor windows may be positioned at different heights from a base of the elongate ridge element. In some embodiments, the plurality of sensor windows may be directed at different angles from one another with respect to a base of the elongate ridge element.

In some embodiments, the elongate ridge element may comprise a substantially triangular prism-shaped element.

The elongate ridge element may, in some embodiments, comprise a central recessed portion, such that a height of the elongate ridge element at its center is smaller than a height of the elongate ridge element at its ends.

In some embodiments, the measurement element may further comprise a cleaning element located within the elongate ridge element.

The measurement element may further comprise a surface from which the elongate ridge element extends.

In some embodiments, the first sensing region may be configured to receive radiation to be delivered to an optical sensor.

The measurement element may, in some embodiments, comprise, or form part of at least one of: a toothbrush head, an interproximal irrigation attachment, and a tongue cleaning attachment.

According to a second aspect, the invention provides a measurement element for an oral care device comprising:
at least two elongate ridge elements configured to engage adjacent teeth within an oral cavity; and
a first sensing region having at least one sensor located between the at least two elongate ridge elements, configured to acquire data from gingival tissue within the oral cavity.

According to a third aspect, the invention provides an oral care device comprising a handle portion, and a measurement element as disclosed herein.

According to a fourth aspect, the invention provides a mouthpiece comprising an arch-shaped body for fitting at least partially over a dental arch of a subject, and a measurement element as disclosed herein, the measurement element located on a surface of the arch-shaped body for engaging an interproximal region of the dental arch of the subject.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a schematic perspective view of an example of a measurement element according to various embodiments;
Fig. 2 is a schematic plan view of the measurement element of Fig. 1;
Fig. 3 is a schematic perspective view of a further example of a measurement element according to various embodiments;
Fig. 4 is a schematic plan view of the measurement element of Fig. 3;
Fig. 5 is a schematic perspective view of a further example of a measurement element according to various embodiments;
Fig. 6 is a schematic plan view of a further example of a measurement element according to various embodiments;
Fig. 7 is a schematic perspective of a further example of a measurement element according to various embodiments;
Fig. 8 is a schematic side view of the measurement element of Fig. 7;
Fig. 9 is a schematic illustration of an example of an oral care device according to various embodiments;
Fig. 10 is a perspective view of an example of an attachment for an oral care device, according to various embodiments;
Fig. 11 is a schematic illustration of a further example of an oral care device according to various embodiments; and
Fig. 12 is a perspective view of an example of a mouthpiece according to various embodiments.

### DETAILED DESCRIPTION OF EMBODIMENTS

Embodiments disclosed herein relate to apparatus that can be used to perform measurements on gingival tissue within the oral cavity of a subject. Examples herein refer to use by a person (i.e. a human user). However, such apparatus may alternatively be used to perform measurements on gingival tissue within the oral cavity of a different subject, such as an animal. Gingival tissue, which is also referred to as gum tissue, or gums, is the tissue found in the area around the root of teeth that helps keep the tooth in place. Gum tissue is also found between teeth.

The measurement element disclosed herein is indicated as being suitable for an oral care device. It is envisaged that such a measurement element may be incorporated into an existing oral care device, such as a power toothbrush, an oral irrigation device, or a tongue cleaning device. Equally, however, it is envisaged that such a measurement element may be incorporated into a device whose primary purpose is to perform measurements of gingival tissue within the oral cavity. As will become apparent, is also envisaged that such a measurement element may be supplemented with additional oral care means, so that a measurement device incorporating such a measurement element may additionally be used to perform oral care tasks, such as interproximal cleaning.

The inventors of the presently-disclosed embodiments have identified that, in order to take accurate measurements of gingival tissue within an oral cavity, a measurement device should be positioned and oriented in a particular manner, and that such positioning an orientation may be difficult for a user to achieve, even if a mirror is used. Therefore, the embodiments presented herein provide a measurement element having a structure that allows for easy and intuitive positioning of the measurement element relative to the gingival tissue from which data is to be acquired.

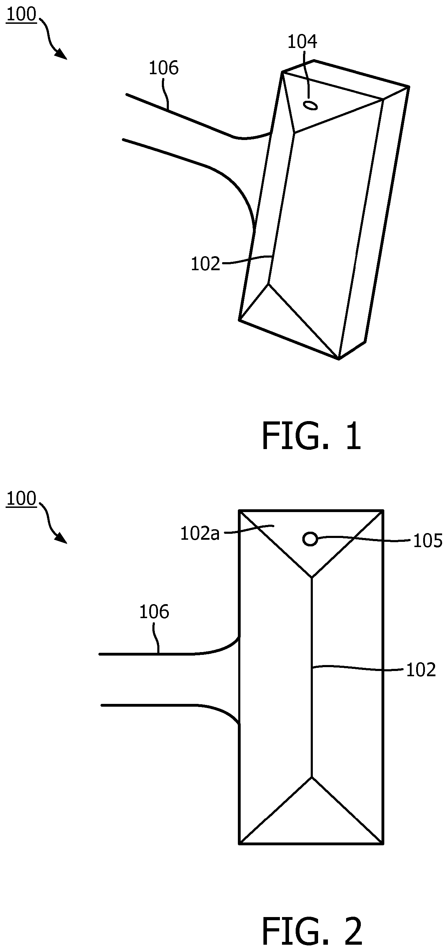

Referring to the drawings, Figs. 1 and 2 show, respectively, a simplified perspective illustration and a simplified plan view illustration of an example of a measurement element 100 for an oral care device. The measurement element 100 comprises at least one elongate ridge element 102 for engaging an interproximal region within an oral cavity. An interproximal region (also referred to as an interproximal space) is the volume or space between adjacent teeth in a person's oral cavity. The elongate ridge element 102 is shaped such that a portion of the ridge element is able to enter the interproximal region when the measurement element 100 is positioned appropriately (e.g. with the longitudinal axis of the ridge element aligned with the interproximal space, or interface between two adjacent teeth, and with the elongate ridge element touching the two adjacent teeth). The measurement element 100 also comprises at least a first sensing region 104. In the arrangement shown in Fig. 1, the first sensing region 104 is located in a first end portion of the elongate ridge element 102 for acquiring data from gingival tissue within the oral cavity.

The elongate ridge element 102 allows the measurement element 100 to remain in a stable orientation between two adjacent teeth (i.e. in the interproximal region) while a measurement is made. According to embodiments disclosed herein, the elongate ridge element 102 may comprise a substantially triangular prism-shaped element, as shown in the drawings. In some embodiments, the ends of the triangular prism-shaped element may be inclined relative to its base, as shown, for example, in Fig. 1. A triangular prism-shaped element has a ridge at its apex which may be at least partially received in an interproximal space between adjacent teeth. In other embodiments, the elongate ridge element 102 may be shaped differently. For example, the ridge element 102 may have some other prismatic structure. In some embodiments, the edges of the elongate ridge element 102 may be curved or chamfered so as to improve the comfort of a user when the measurement element 100 is pressed against a surface in the oral cavity. Moreover, curved edges may help to prevent injury to oral tissue.

By having a first sensing region 104 located in a first end portion 102a of the ridge element 102, when the ridge element is in the intended position, the sensing region will be positioned appropriately to acquire data from the gingival tissue. Thus, a user of the measurement element 100 does not need to be too concerned with the position of the sensing region 104 because, once the ridge element 102 is lodged between two teeth, the sensing region 104 will be positioned appropriately, facing the gingiva in the interproximal region.

The first sensing region 104 may comprise a sensor 105 positioned on a surface of, or at least partially within, the elongate ridge element 102. In some embodiments, a sensor 105 may be located on or in an end wall 102a or face of the ridge element 102, for example. In other embodiments, the sensing region 104 may comprise a window or aperture through which sensing may be performed. For example, the first sensing region 104 shown in Fig. 1 may comprise a sensor window. A sensor 105 which is configured to receive a signal (e.g. a reflected optical signal) from the target gingiva may be located within the elongate ridge element 102, or elsewhere within the measurement element 100 or within an associated oral care device. In such examples, a conduit, such as an optical waveguide (e.g. an optical fiber) may deliver an acquired signal from the sensing region 104 (e.g. a sensor window) to the sensor.

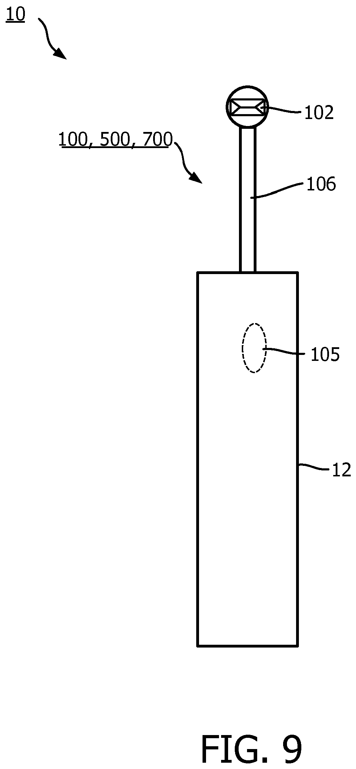

Thus, in some embodiments, the first sensing region 104 may be configured to receive radiation to be delivered to an optical sensor 105. The optical sensor may, for example, be located within the measurement element 100 or elsewhere, such as within a body of an associated oral cleaning device 10, such as shown in Fig. 9. In other embodiments, other types of sensors may be used. For example, the first sensing region 104 may comprise one or more of the following types of sensors: a proximity sensor, an electrical impedance sensor, a radiofrequency (RF) sensor, a terahertz sensor, an ultrasound sensor, or a microphone.

Figs. 1 and 2 also show a portion of a neck 106, which may be provided to enable the measurement element 100 to be mounted to a body of an oral care device 10, for example, such as shown in Fig 9..

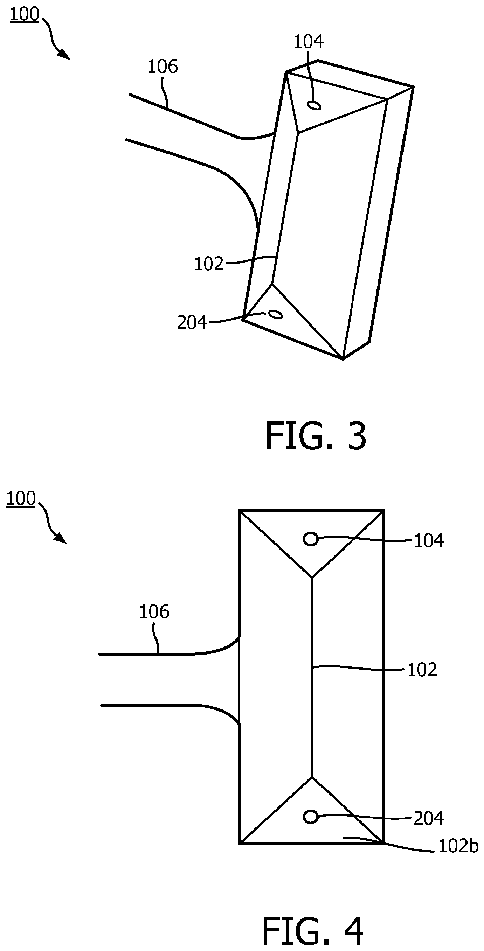

Figs. 3 and 4 show, respectively, a simplified perspective illustration and a simplified plan view illustration of a further example of a measurement element 100 for an oral care device. The measurement element 100 shown in Figs. 3 and 4 includes the elongate ridge element 102 and the first sensing region 104, and further comprises a second sensing region 204 located in a second end portion 102b of the elongate ridge element 102 for acquiring data from gingival tissue within the oral cavity. The second sensing region 204 may be the same as, or similar to, the first sensing region 104 and, as discussed above with reference to the first sensing region, may comprise a window or aperture through which sensing may be performed. A signal received through such a window may be delivered (e.g. via an optical waveguide) to a sensor 105 located within the ridge element 102 or elsewhere within the measurement element or an associated oral care device 10, such as shown in Fig. 9. In some embodiments, signals received via the first and second sensing regions 104, 204 may be delivered to the same sensor.

By providing a second sensing region 204 in the measurement element 100, a user is able to use the same measurement element for performing measurements in respect of (i.e. capturing data from) the gingiva at the top of the oral cavity and at the bottom of the oral cavity. For example, in the orientation shown in Fig. 3, the measurement element 100 may be used to capture data from gingiva in interproximal regions between teeth in an upper set of a person's teeth (i.e. in an upper dental arch) using the first sensing region 104, and used to capture data from gingiva in interproximal regions between teeth in a lower set of a person's teeth (i.e. in a lower dental arch) using the second sensing region 204.

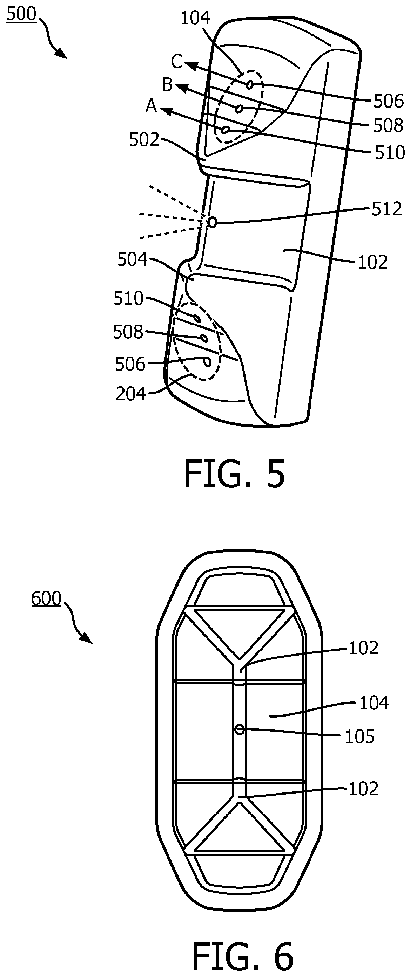

Fig. 5 shows a further example of a measurement element 500 for an oral care device. The measurement element 500 shown in Fig. 5 includes the elongate ridge element 102. In some embodiments, such as that shown in Fig. 5, the measurement element 500 may further comprise a first protrusion 502 extending from the first end portion of the elongate ridge element 102. The first sensing region 104 may be located in a wall of the first protrusion 502. In some embodiments, such as that shown in Fig. 5, the measurement element 500 may further comprise a second protrusion 504 extending from a second end portion of the elongate ridge element 102. The second sensing region 204 may be located in a wall of the second protrusion 504. The protrusions 502, 504 may be considered to be part of the elongate ridge element 102. For example, the protrusions 502, 504 themselves may be considered to comprise the end portions of the elongate ridge element 102.

While the measurement element 500 shown in Fig. 5 is shown to include two protrusions (i.e. the first protrusion 502 and the second protrusion 504) a measurement element may, in other embodiments, include just one protrusion (e.g. the first protrusion 502). Since most teeth in a person's oral cavity are curved, a straight elongate ridge element 102 without a protrusion 502, 504 (e.g. the ridge element of the measurement element 100 shown in Figs. 1 to 4) may rock or pivot when positioned in the interproximal region between two teeth, especially if the teeth are particularly curved. By including a protrusion 502, 504 at both ends of the ridge element 102, a more secure positioning of the measurement element within the interproximal region can be achieved. A single protrusion may improve the stability of the measurement element when held against the teeth, while two protrusions may improve the stability to an even greater extent.

The protrusions 502, 504 may take any form suitable for housing a sensing region. In some embodiments, one or more of the protrusions 502, 504 may comprise a partial pyramidal protrusion or a partial conical protrusion. An example of protrusions having a partial pyramidal shape is shown in Fig. 5. In other embodiments, protrusions may have a different shape or configuration. For example, a pyramid-shaped protrusion or conical protrusion may be used. In general, a protrusion having a substantially pointed apex may allow the measurement element 100, 500 to be positioned more stably within an interproximal region. Edges of the protrusions may be rounded or chamfered to avoid damaging tissue within the oral cavity and to improve the user's comfort. Similarly, the apex and/or edges of the protrusions may be rounded in a similar way.

In the embodiments shown in Figs. 1 to 4, each sensing region 104, 204 comprises a single sensor 105 or sensor window located within an end portion of the elongate ridge element 102. In other embodiments, each sensing region 104, 204 may comprise multiple sensors, sensing elements or sensor windows. In the measurement elements 500 shown in Fig. 5, each sensing region 104, 204 comprises three sensor windows (i.e. a first sensor window 506, a second sensor window 508 and a third sensor window 510) arranged at different heights up the side of an end wall of the protrusions 502, 504. More generally, each sensing region 104, 204 may comprise a plurality of sensor windows. The plurality of sensor windows may be positioned at different heights from a base of the elongate ridge element 102. An advantage of arranging a plurality of sensor windows 506, 508, 510 at a plurality of different heights with respect to the base of the elongate ridge element 102 is that, when the measurement element 500 is positioned appropriately within the oral cavity (e.g. with the apex of the ridge element and/or the apex of each protrusion 502, 504 engaged in an interproximal region), at least one of the sensor windows is likely to be in a suitable position for receiving a signal (e.g. a reflected optical signal) from the gingiva.

In embodiments in which multiple sensor windows are included at each sensing region 104, 204 (e.g. the embodiment shown in Fig. 5) one or more of the sensor windows 506, 508, 510 may be aimed in a different direction to the other sensor windows. For example, one or more of the sensor windows 506, 508, 510 may be directed at an angle with respect to the base of the elongate ridge element 102 which is different to the angle at which another of the sensor windows is directed. More generally, each sensing region 104, 204 may comprise a plurality of sensor windows. The plurality of sensor windows may be directed at different angles from one another with respect to a base of the elongate ridge element 102. In the example shown in Fig. 5, the difference in the directional angles is shown by arrows labelled A, B and C. For example, the first sensor window 506 is aimed or directed in a direction indicated by arrow A, the second sensor window 508 is aimed or directed in a direction indicated by arrow B and the third sensor window 510 is aimed or directed in a direction indicated by arrow C. By arranging the sensor windows in this way, there is an increased likelihood that at least one of the sensor windows will be appropriately oriented and/or aligned with respect to the gingiva, when the measurement element 500 is positioned in the oral cavity.

In some embodiments, each sensor window 506, 508, 510 may serve to direct a signal (e.g. an optical signal) out from the measurement element 500, and receive a reflected signal through the same sensor window. In other embodiments, a signal may be directed out of one of the sensor windows, and a reflected signal may be received through another of the sensor windows. In some embodiments, signals may be directed out of all of the sensor windows in a sensing region, and each of the sensor windows may also receive a reflected signal.

It will be appreciated that each sensor window 506, 508, 510 may have a different size and/or shape. In some embodiments, however, all of sensor windows may have the same size and/or shape.

In some embodiments, the measurement element may function in conjunction with a cleaning element, such as an oral cleaning element. For example, in some embodiments, the measurement element may include a cleaning element while, in other embodiments, a cleaning element (e.g. a cleaning element of an oral cleaning device) may incorporate a measurement element as disclosed herein. In some examples, the measurement element may comprise a cleaning element located within the elongate ridge element 102. An example of such a cleaning element incorporated into the elongate ridge element 102 is shown in the embodiments shown in Fig. 5. In Fig. 5, a cleaning element 512 includes an aperture or opening in the elongate ridge element 102. In these embodiments, the aperture is formed in the apex of the ridge element; however, it will be appreciated that such a cleaning element may be positioned in the measurement element and/or elsewhere in the ridge element. In this example, the cleaning element aperture 512 is configured to direct gas (e.g. air), liquid (e.g. water), or a combination of gas and liquid from a source located within the ridge element, the measurement element, or an associated oral cleaning device, towards a target in the user's oral cavity. For example, a combination of water and air may be ejected through the aperture 512 (e.g. as shown by the dashed lines in Fig. 5) into an interproximal region to provide a cleaning effect between the teeth. In other embodiments, the cleaning element 512 may comprise a bristle or tuft of bristles, for example, to engage with and clean the interproximal region while the measurement element 500 is in position in the oral cavity. In other embodiments, another type of cleaning element may be incorporated into the measurement element 500.

While the cleaning element 512 is shown only in the measurement elements 500, it will be appreciated that such a cleaning element may be incorporated into any of the embodiments discussed herein.

Fig. 6 shows another arrangement of a measurement device 600 which has ridge elements 102 at the outer ends, with a sensing region 104 located between the ridge elements that projects out beyond the ridge elements. This arrangement enables the ridge elements 102 to be positioned on adjacent teeth such that the sensing region 104 projects into the interproximal region between the two teeth. The sensing region 104 shown in Fig. 6 comprises a single sensor 105 or sensor window and the sensing region 104 is located between the two or more elongate ridge elements 102. However, the sensing region 104 may comprise multiple sensors, sensing elements or sensor windows. An advantage of arranging the sensing region 104 at a different height with respect to the base of the elongate ridge element 102 is that, when the sensing region 104 is positioned appropriately within the oral cavity (e.g. with the sensor 105 engaged in an interproximal region), it is likely to be in a suitable position for receiving a signal (e.g. a reflected optical signal) from the gingiva.

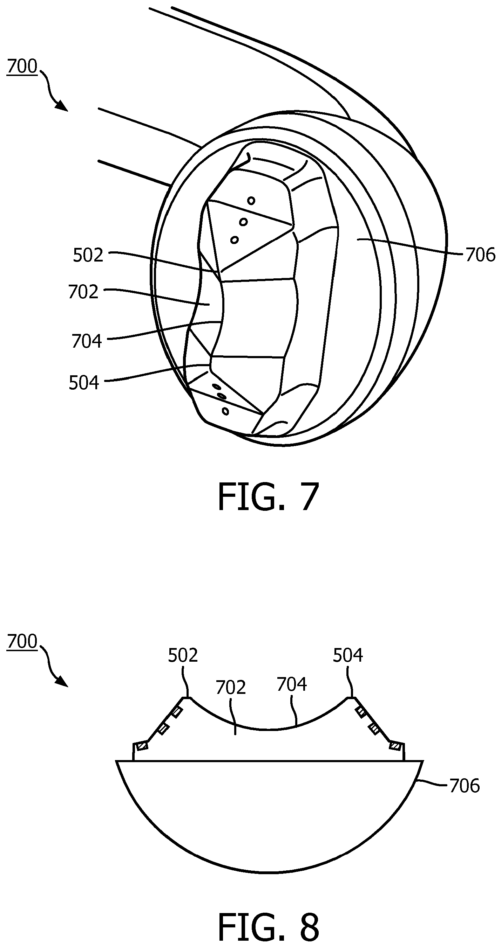

Fig. 7 is a schematic perspective view of a further example of a measurement element 700 for an oral care device. The measurement element 700 is similar to the measurement element 500 shown in Fig. 5. However, the measurement element 700 includes a number of additional features.

In the embodiments discussed above, the elongate ridge element comprises a linear ridge (i.e. the apex of the ridge element 102). In other words, in the embodiments discussed above, the height of the ridge of the ridge element 102 above its base is approximately constant along its length. However, in some embodiments, the elongate ridge element may comprise a central recessed portion, such that a height of the elongate ridge element at its centre is smaller than a height of the elongate ridge element at its ends. Such an arrangement is shown in the example embodiment of the measurement element 700 in Fig. 7. The measurement element 700 includes an elongate ridge element 702 which has, at its ends, the first protrusion 502 and a second protrusion 504. In this arrangement, however, the ridge of elongate ridge element 702 dips (e.g. reduces in height) between the first and second protrusions, thereby forming a central recessed portion 704.

Fig. 8 is a schematic side view of the measurement element 700 shown in Fig. 7. From the side view, the central recessed portion 704 can be seen clearly between the first and second protrusions 502, 504. While, in the embodiment shown, the central recessed portion 704 is curved, in other embodiments, the elongate ridge element may include a stepped portion in which the height of the elongate ridge element is reduced in a central region. An advantage of including the central recessed portion 704 in the elongate ridge element 702, particularly one formed with a curved ridge (e.g. as shown in Figs. 7 and 8), is that the measurement element is able to move more easily over relatively short teeth (e.g. the lower incisors) and is able to move more easily over bumps in the oral cavity and on the teeth. It will be appreciated that the central recessed portion 704 may be incorporated into any of the measurement elements discussed herein.

In some embodiments, the measurement element may comprise a surface from which the elongate ridge element 702 extends. For example, the measurement element 700 includes a surround 706, which provides a surface from which the elongate ridge element and the protrusions of the measurement element may be formed or mounted. In some embodiments, the elongate ridge element 702, the protrusions 502, 504 and the surround 706 may be formed as a single, integral unit while, in other embodiments, the elongate ridge element and the protrusions may form a separate component to be mounted onto the surround. **In** some embodiments, the surround 706 may be substantially circular in shape while, in other embodiments, the surround may be shaped differently, for example as an oval. The surround 706 may aid the user in positioning and orienting the measurement element appropriately within the oral cavity, as the surround may prevent or restrict rotation of the measurement element when the surround engages a tooth. A smaller surround 706 (e.g. an oval-shaped surround) may assist the user in positioning the measurement element appropriately in particularly curved regions of the oral cavity (e.g. near the canine teeth). It will be appreciated that any of the measurement elements disclosed herein may include a surround, such as the surround 706, such that the elongate ridge element extends from a surface of the surround. Moreover, it will be appreciated that any of the features or combination of the features disclosed herein may be incorporated into any of the disclosed embodiments.

**In** some embodiments, the surround 706 may include other components, such as oral cleaning elements 512, such as shown in Fig. 5. For example, the surround 706 may include one or more bristles or tuft of bristles, one or more interproximal irrigation elements (not shown in Figs. 7 and 8) for directing gas and/or water into an interproximal region, and/or one or more tongue cleaning elements. **In** this way, the measurement element may be considered to include an oral cleaning element.

Thus, the measurement element may, in some embodiments, comprise, or form part of at least one of a toothbrush head, an interproximal irrigation head or attachment, and a tongue cleaning head or attachment. As used herein, the term "attachment" is considered to refer to a part of an oral cleaning device which, for example, can be removed from and reattached to a body of the oral cleaning device.

According to a second aspect, the invention provides an oral care device 10. Fig. 9 is a schematic illustration of an example of an oral care device 10 according to various embodiments. The oral care device 10 comprises a handle portion 12 and a measurement element 100, 500, 700, as described herein. **In** some embodiments, the oral care device 10 may be considered to be an oral measurement device having at least one sensing region 104, 204, such that its primary function is to perform measurements on gingiva within the oral cavity. **In** the embodiment shown in Fig. 9, the oral care device 10 includes a handle portion 12, the measurement element 100, 500, 700, and a neck portion 106 connecting the measurement element to the handle portion. **In** the arrangement shown in Fig.9, the sensor 105 for the measurement element 100, 500, 700 is shown in the handle portion 12. It can be appreciated that the sensor 105 can be located in the measurement element 100, 500, 700, in the handle 12, or elsewhere. It will also be appreciated that, in other embodiments, the measurement element 100, 500, 700 may form part of the handle portion 12, or may be connected directly to it (i.e. without a neck portion). The oral care device 10 may include other features, such as electrical components, a power button, and the like, but these are omitted from Fig. 9 for clarity.

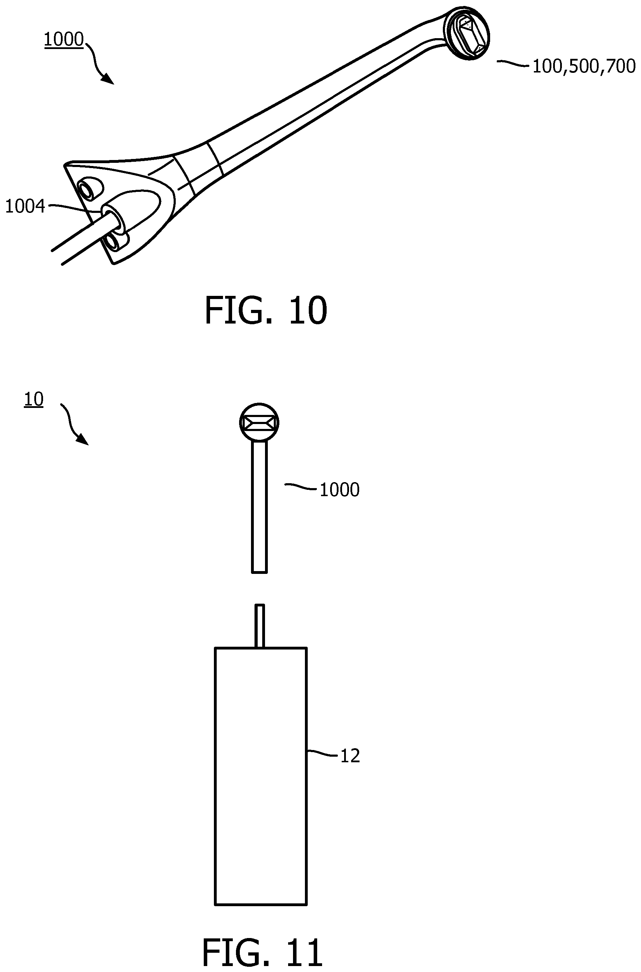

According to a third aspect, the invention provides an attachment for an oral care device 10. Fig. 10 is a perspective view of an example of an attachment 1000 for an oral care device 10, according to various embodiments. The attachment 1000 comprises a measurement element 100, 500, 700 and a connection portion 1004 for connecting the attachment to a handle portion 12of the oral care device, as shown in Fig. 11. The connection portion 1004 may include any suitable connection mechanism for attaching the attachment 1000 to a handle 12 of an oral care device 10. Such connection mechanisms will be the known to those skilled in the field.

Fig. 11 is a schematic illustration of a further example of an oral care device 10 according to various embodiments. The oral care device 10 comprises a handle portion 12 and an attachment 1000. The attachment may comprise the attachment 1000 discussed above. The attachment 1000 may connect or attach to the handle portion 12 using the connection portion 1004 which may, for example, interact with a complementary connection mechanism of the handle portion 12.

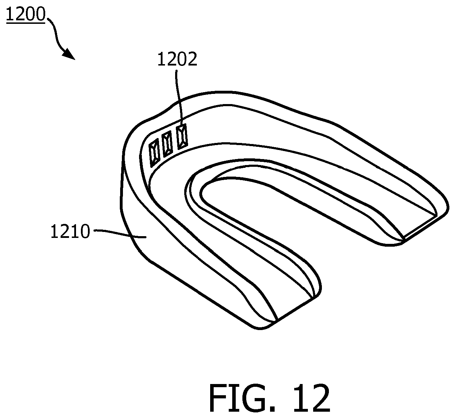

According to another aspect, the invention provides a mouthpiece. Fig. 12 is a perspective view of an example of a mouthpiece 1200 according to various embodiments. The mouthpiece 1200 comprises an arch-shaped body 1210 for fitting at least partially over a dental arch of a subject, and a measurement element 1202. The measurement element 1202 is located on an interior surface of the arch-shaped body 1210 for engaging an interproximal region of the dental arch of the subject. The measurement element 1202 may comprise a measurement element as described herein (e.g. measurement elements 100, 500, 700). In some embodiments, the mouthpiece 1200 may comprise a single measurement element 1202. In another embodiment, however, the mouthpiece 1200 may comprise multiple measurement elements 1202 spaced around the arch-shaped body 1210. Each measurement element 1202 may be arranged to engage an interproximal region between a different adjacent pair of teeth. In the embodiment shown in Fig. 12, the mouthpiece 1200 includes three measurement elements 1202. However, it will be appreciated that more or fewer measurement elements may be included. The mouthpiece may be worn by a user and, when the measurement elements are positioned appropriately, data relating to the gingiva may be acquired by each measurement element.

Variations to the disclosed embodiments can be understood and effected by those skilled in the art in practicing the claimed invention, which is defined by the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A measurement element (100) for an oral care device (10), the measurement element comprising:
at least one elongate ridge element (102) configured to engage an interproximal region within an oral cavity; and
a first sensing region (104) located in a first end portion (102a) of the at least one elongate ridge element, configured to acquire data from gingival tissue within the oral cavity.

2. A measurement element (100, 500) according to claim 1, further comprising:
a first protrusion (502) extending from the first end portion of the at least one elongate ridge element;
wherein the first sensing region (104) is located in a wall of the first protrusion.

3. A measurement element (100, 500) according to claim 1 or claim 2, further comprising:
a second sensing region (204) located in a second end portion (102b) of at least one elongate ridge element (102) for acquiring data from gingival tissue within the oral cavity.

4. A measurement element (100, 500) according to claim 3, further comprising:
a second protrusion (504) extending from a second end portion of the at least one elongate ridge element (102);
wherein the second sensing region (204) is located in a wall of the second protrusion.

5. A measurement element (100, 500) according to any of the preceding claims, wherein each sensing region (104, 204) comprises a plurality of sensor windows (506, 508, 510); and
wherein the plurality of sensor windows are at least one of:
a) positioned at different heights from a base of the at least one elongate ridge element (102); and
b) directed at different angles from one another with respect to a base of the at least one elongate ridge element.

6. A measurement element (100, 500) according to any of the preceding claims, wherein the at least one elongate ridge element (102) comprises a substantially triangular prism-shaped element.

7. A measurement element (100, 500, 700) according to any of the preceding claims, wherein the at least one elongate ridge element (102) comprises a central recessed portion, (704), such that a height of the at least one elongate ridge element at its center is smaller than a height of the at least one elongate ridge element at its ends.

8. A measurement element (100, 500, 700) according to any of the preceding claims, further comprising:
a cleaning element (512) located within the at least one elongate ridge element (102).

9. A measurement element (100, 500, 700) according to any of the preceding claims, further comprising a surface (706) from which the at least one elongate ridge element (702) extends.

10. A measurement element (100) according to any of the preceding claims, wherein the first sensing region (104) is configured to receive radiation to be delivered to an optical sensor (105).

11. A measurement element (600) for an oral care device (10) comprising:
at least two elongate ridge elements (102) configured to engage adjacent teeth within an oral cavity; and
a first sensing region (104) having at least one sensor (105) located between the at least two elongate ridge elements, configured to acquire data from gingival tissue within the oral cavity.

12. An oral care device (10) comprising:
a handle portion (12); and
a measurement element (100,500,600,700) according to any of the preceding claims.

13. A mouthpiece (1200) comprising:
an arch-shaped body (1210) for fitting at least partially over a dental arch of a subject; and
a measurement element (1202) according to any of claims 1 to 11, the measurement element located on a surface of the arch-shaped body for engaging an interproximal region of the dental arch of the subject.

## Patentansprüche

1. Messelement (100) für ein Mundpflegegerät (10), wobei das Messelement Folgendes umfasst:
mindestens ein längliches Leistenelement (102), das dazu konfiguriert ist, mit einem interproximalen Bereich innerhalb einer Mundhöhle in Eingriff zu kommen; und
einen ersten Erfassungsbereich (104), der sich in einem ersten Endabschnitt (102a) des mindestens einen länglichen Leistenelements befindet, dazu konfiguriert, Daten vom gingivalen Gewebe innerhalb der Mundhöhle zu erfassen.

2. Messelement (100, 500) nach Anspruch 1, weiter umfassend:
einen ersten Vorsprung (502), der sich vom ersten Endabschnitt des mindestens einen länglichen Leistenelements erstreckt;
wobei sich der erste Erfassungsbereich (104) in einer Wand des ersten Vorsprungs befindet.

3. Messelement (100, 500) nach Anspruch 1 oder Anspruch 2, weiter umfassend:
einen zweiten Erfassungsbereich (204), der sich in einem zweiten Endabschnitt (102b) mindestens eines länglichen Leistenelements (102) befindet, um Daten vom gingivalen Gewebe innerhalb der Mundhöhle zu erfassen.

4. Messelement (100, 500) nach Anspruch 3, weiter umfassend:
einen zweiten Vorsprung (504), der sich von einem zweiten Endabschnitt des mindestens einen länglichen Leistenelements (102) erstreckt;
wobei sich der zweite Erfassungsbereich (204) in einer Wand des zweiten Vorsprungs befindet.

5. Messelement (100, 500) nach einem der vorstehenden Ansprüche,
wobei jeder Erfassungsbereich (104, 204) eine Vielzahl von Sensorfenstern (506, 508, 510) umfasst; und
wobei die Vielzahl von Sensorfenstern mindestens eines des Folgenden ist:
a) in unterschiedlichen Höhen von einer Basis des mindestens einen länglichen Leistenelements (102) positioniert; und
b) in Bezug auf eine Basis des mindestens einen länglichen Leistenelements in unterschiedlichen Winkeln zueinander ausgerichtet.

6. Messelement (100, 500) nach einem der vorstehenden Ansprüche,
wobei das mindestens eine längliche Leistenelement (102) ein im Wesentlichen dreieckiges, prismenförmiges Element umfasst.

7. Messelement (100, 500, 700) nach einem der vorstehenden Ansprüche, wobei das mindestens eine längliche Leistenelement (102) einen zentralen vertieften Abschnitt (704) umfasst, sodass eine Höhe des mindestens einen länglichen Leistenelements in seiner Mitte kleiner ist als eine Höhe des mindestens einen länglichen Leistenelements an dessen Enden.

8. Messelement (100, 500, 700) nach einem der vorstehenden Ansprüche, weiter umfassend:
ein Reinigungselement (512), das sich innerhalb des mindestens einen länglichen Leistenelements (102) befindet.

9. Messelement (100, 500, 700) nach einem der vorstehenden Ansprüche, das weiter eine Oberfläche (706) umfasst, von der sich das mindestens eine längliche Leistenelement (702) erstreckt.

10. Messelement (100) nach einem der vorstehenden Ansprüche,
wobei der erste Erfassungsbereich (104) dazu konfiguriert ist, Strahlung zu empfangen, die an einen optischen Sensor (105) weitergeleitet werden soll.

11. Messelement (600) für ein Mundpflegegerät (10), umfassend:
mindestens zwei längliche Leistenelemente (102), die dazu konfiguriert sind, mit angrenzenden Zähnen in einer Mundhöhle in Eingriff zu kommen; und
einen ersten Erfassungsbereich (104), der mindestens einen Sensor (105) aufweist, der zwischen den mindestens zwei länglichen Leistenelementen befindet, dazu konfiguriert, Daten vom gingivalen Gewebe innerhalb der Mundhöhle zu erfassen.

12. Mundpflegegerät (10), umfassend:
einen Griffabschnitt (12); und
ein Messelement (100, 500, 600, 700) nach einem der vorstehenden Ansprüche.

13. Mundstück (1200), umfassend:
einen bogenförmigen Körper (1210), der mindestens teilweise über einen Zahnbogen eines Subjekts passt; und
ein Messelement (1202) nach einem der Ansprüche 1 bis 11, wobei sich das Messelement auf einer Oberfläche des bogenförmigen Körpers befindet, um mit einem interproximalen Bereich des Zahnbogens des Subjekts in Eingriff zu kommen.

## Revendications

1. Élément de mesure (100) pour dispositif de soins bucco-dentaires (10), l'élément de mesure comprenant :
au moins un élément arête allongé (102) configuré pour venir en prise avec une région interproximale à l'intérieur d'une cavité buccale ; et
une première région de détection (104) située dans une première partie d'extrémité (102a) du au moins un élément arête allongé, configurée pour acquérir des données à partir du tissu gingival dans la cavité buccale.

2. Élément de mesure (100, 500) selon la revendication 1, comprenant en outre :
une première saillie (502) s'étendant à partir de la première partie d'extrémité du au moins un élément arête allongé ;
dans lequel la première région de détection (104) est située dans une paroi de la première saillie.

3. Élément de mesure (100, 500) selon la revendication 1 ou la revendication 2, comprenant en outre :
une seconde région de détection (204) située dans une seconde partie d'extrémité (102b) d'au moins un élément arête allongé (102) pour acquérir des données à partir du tissu gingival dans la cavité buccale.

4. Élément de mesure (100, 500) selon la revendication 3, comprenant en outre :
une seconde saillie (504) s'étendant à partir d'une seconde partie d'extrémité du au moins un élément arête allongé (102) ;
dans lequel la seconde région de détection (204) est située dans une paroi de la seconde saillie.

5. Élément de mesure (100, 500) selon l'une quelconque des revendications précédentes,
dans lequel chaque région de détection (104, 204) comprend une pluralité de fenêtres de détection (506, 508, 510) ; et
dans lequel la pluralité de fenêtres de détection sont d'au moins un type parmi :
a) positionnées à différentes hauteurs par rapport à une base du au moins un élément arête allongé (102) ; et
b) orientées selon des angles différents les unes des autres par rapport à une base du au moins un élément arête allongé.

6. Élément de mesure (100, 500) selon l'une quelconque des revendications précédentes,
dans lequel le au moins un élément arête allongé (102) comprend un élément en forme de prisme sensiblement triangulaire.

7. Élément de mesure (100, 500, 700) selon l'une quelconque des revendications précédentes, dans lequel le au moins un élément arête allongé (102) comprend une partie centrale en retrait (704), de sorte qu'une hauteur du au moins un élément arête allongé en son centre soit inférieure à une hauteur du au moins un élément arête allongé à ses extrémités.

8. Élément de mesure (100, 500, 700) selon l'une quelconque des revendications précédentes, comprenant en outre :
un élément de nettoyage (512) situé à l'intérieur du au moins un élément arête allongé (102).

9. Élément de mesure (100, 500, 700) selon l'une quelconque des revendications précédentes, comprenant en outre une surface (706) de laquelle s'étend le au moins un élément arête allongé (702).

10. Élément de mesure (100) selon l'une quelconque des revendications précédentes,
dans lequel la première région de détection (104) est configurée pour recevoir un rayonnement à émettre vers un capteur optique (105).

11. Élément de mesure (600) pour dispositif de soins bucco-dentaires (10) comprenant :
au moins deux éléments arêtes allongés (102) configurés pour venir en prise avec des dents adjacentes dans une cavité buccale ; et
une première région de détection (104) présentant au moins un capteur (105) situé entre les au moins deux éléments arêtes allongés, configuré pour acquérir des données à partir du tissu gingival dans la cavité buccale.

12. Dispositif de soins bucco-dentaires (10), comprenant :
une partie de manche (12) ; et
un élément de mesure (100, 500, 600, 700) selon l'une quelconque des revendications précédentes.

13. Embout buccal (1200) comprenant :
un corps arqué (1210) destiné à s'adapter au moins partiellement sur une arcade dentaire d'un sujet ; et
un élément de mesure (1202) selon l'une quelconque des revendications 1 à 11, l'élément de mesure étant situé sur une surface du corps arqué pour venir en prise avec une région interproximale de l'arcade dentaire du sujet.
